Europäisches Patentamt

European Patent Office

Office européen des brevets

(19)

(11) Publication number: **0 182 296**
**A2**

(12) # EUROPEAN PATENT APPLICATION

(21) Application number: **85114491.5**

(22) Date of filing: **14.11.85**

(51) Int. Cl.⁴: **A 61 K 7/035,** C 12 P 19/14

(30) Priority: **15.11.84 US 671701**

(43) Date of publication of application: **28.05.86**
**Bulletin 86/22**

(84) Designated Contracting States: **BE CH DE FR GB IT LI
NL**

(71) Applicant: **CPC INTERNATIONAL INC., International
Plaza P.O. Box 8000, Englewood Cliffs New
Jersey 07632 (US)**

(72) Inventor: **Kochan, David A., 1534 - 77th Street, Darien
Illinois 60559 (US)**
Inventor: **Zobel, Henry F., 1105 Belair Drive, Darien
Illinois 60559 (US)**

(74) Representative: **Lederer, Franz, Dr. et al, Patentanwälte
Dr. Franz Lederer Dipl.-Ing. Reiner F. Meyer-Roxlau
Lucile-Grahn-Strasse 22, D-8000 München 80 (DE)**

(54) Starch for the use in dusting powders.

(57) A dusting powder composition containing porous starch
granules is prepared by treating granular starch with an alpha-
amylase enzyme. The enzyme treatment is carried out under
conditions which solubilize a portion of the starch while leaving
a porous granular starch residue.

ACTORUM AG

3333

## STARCH FOR THE USE IN DUSTING POWDERS

This invention relates to a porous granular starch product and a process for its manufacture. The starch, useful as a dusting powder, is the insoluble residue which remains after a granular starch is partially solubilized by an enzyme.

There is today a need for a material which can serve as a base for body dusting powders. Generally, a widely-used constituent of such formulations has been talc. However, talc has come under increasing medical scrutiny as possibly containing a carcinogenic substance, asbestos. Pure mineral talc is a group of hydrous magnesium silicates. Unlike pure mineral talc, commercial talc has varying compositions depending on the source and method of production. It has been postulated that some of the commercial talc may be contaminated with asbestos.

Commercial native corn starch has been used to some extent as a replacement for talc in body dusting powders, particularly, in baby powder. However, unmodified corn starch is not completely satisfactory for these purposes. When it absorbs moisture during use, the granules become tacky and adhere to each other. This results in a gummy, pasty feel on the skin and the moistened starch can form rolls or beads of starch rather than spreading evenly.

Numerous attempts have been made to prepare modified starches that would have good tactile properties even in the presence of moisture. One such product was disclosed by Krisan in U.S. Patent 2,614,945. In this disclosure, starch granules were coated with a thin layer of an insoluble inorganic salt. In U.S. Patent 2,852,404, Satterthwaite disclosed coating starch granules with an aluminum soap. Other workers have proposed making inhibited starches, such as cross-linked starches or cross-linked esters for use as dusting powders. While such procedures do give compositions which are more hydrophobic than native starch, the products generally have poor tactile properties or do not sorb sufficient moisture to make them suitable for many dusting starch uses. In particular, they are not suitable as body dusting starches.

0182296

In U.S. Patent 3,922,199, Hebeda, et al disclose a process for converting granular starch to a soluble hydrolyzate wherein the unconverted starch retains its granular nature. The starch is solubilized by the action of alpha-amylase at a temperature below the actual gelatinization temperature of the starch. We have now discovered that such a granular starch residue has good tactile properties when used as a body dusting starch. It sorbs much more water than either talc or native starch.

In accordance with this invention, there is provided a dusting powder comprising porous starch granules capable of sorbing from about 1.2 grams to about 2.5 grams of water per gram of starch, said porous starch granules comprising the insoluble residue remaining after granular starch is partially solubilized by an alpha-amylase enzyme alone or in combination with glucoamylase.

Also provided, in accordance with this invention, is a process for preparing a dusting powder starch, which comprises agitating a mixture of an aqueous dispersion of granular starch and an alpha-amylase enzyme preparation at a temperature where at least part of the granular structure of the starch is retained and at a pH of from about 4.0 to about 7.0, maintaining said conditions for a sufficient time to solubilize from about 45% to about 90% by weight of the granular starch, separating porous granular starch residue from the reaction mixture and washing the porous granular starch to remove adhering material.

-3-

The improved dusting starch of this invention consists primarily of granular starch which has been treated with an alpha-amylase enzyme. Treatment is carried out in such a manner that at least part of the granular structure of the starch is retained. The resulting product comprises starch granules with varying degrees of porosity as observed under a microscope. The pores in the granules range from about 1 to 4 microns in diameter. The product has a much lower bulk density than that of native starch. The dusting powder of this invention sorbs up to three times as much water as the same weight of talc or native starch sorbs under the same conditions.

All types of starches are suitable as starting material for this invention. For example, both cereal and root starches, such as corn, milo, wheat, rice, arrowroot, potato, tapioca, waxy corn and waxy milo are suitable. The starting starch material may also be a modified starch. Suitable modified starches include oxidized starch, starch esters and starch ethers.

While the type of starch used as a starting material is not critical, it is important that it be in a granular and ungelatinized form. As is well known, native starch exists in the form of granules which exhibit a characteristic birefringence when viewed under a polarizing microscope. When a mixture of starch and water is heated, the granules lose this birefringence and begin to swell. These changes occur over a temperature range known as the gelatinization temperature range. For corn starch, this range is about 62$^o$C to 72$^o$C.

In the process of this invention, the temperature of the starch slurry is initially held slightly below the gelatinization temperature range of the starch. The temperature is so controlled that at least part of the granular structure of the starch is retained during the treatment with alpha-amylase enzyme. It has been found that the permissible temperature increases somewhat during the course of the reaction. In general, the suitable temperature increases from about 60$^o$C to about 75$^o$C during the treatment of corn starch.

The dusting powder starch of the present invention is, as noted above, prepared by treating an ordinary granular starch with an alpha-amylase enzyme. The alpha-amylase used is preferably a bacterial alpha-amylase which is active within the pH range of from about 4.0 to about 7.0.  Preferred sources of such alpha-amylases include several species of the bacillus microorganism, such as Bacillus subtilis, Bacillus licheniformis, Bacillus coagulans, Bacillus amyloliquefaciensH and Bacillus stearothermophilus. Preferred alpha-amylases are the thermostable alpha-amylases produced by Bacillus stearothermophilus and Bacillus licheniformis. For such use, the alpha-amylase is used in a concentration ranging from about 1 unit to about 25 units per gram of starch (dry basis). A preferred range of concentration of alpha-amylase is from about 1.0 to about 10 units per gram of starch (dry basis).

The alpha-amylase activity of an enzyme preparation is determined by the following process.

The solution to be analyzed is diluted with 0.0025 M calcium chloride solution to give a final concentration of about 0.25 unit of activity per ml.  One ml of properly diluted enzyme solution is added to 10 ml of a 1% soluble starch solution containing 0.03 M acetic acid buffer (pH 6.0) and 0.03 M calcium chloride.  The reaction is carried out for 10 minutes at 60°C. One ml of the reaction solution is put in a 100-ml graduated flask containing 50 ml of 0.02 $\underline{N}$ hydrochloric acid, and after adding 3 ml of 0.05% iodine solution thereto, the total volume is made up to

-6-

100 ml by the addition of water. The blue color which develops is measured for absorbance at 620 nm. The amount of the enzyme required to decompose 10 mg/starch in 1 minute is defined as 1 unit.

$$1 \text{ unit} = \frac{D_o - D_s}{D_o} \times \frac{50}{10 \times 10} \times \text{(dilution factor)}$$

where,

$D_o$ = absorbance of control solution (water is added instead of the enzyme solution)

$D_s$ = absorbance of the reaction solution

In the process of this invention, the aqueous dispersion of granular starch is treated with the <u>alpha</u>-amylase enzyme preparation at a pH of from about 4.0 to about 7.0. When the <u>alpha</u>-amylase enzyme preparations are obtained from <u>Bacillus</u> <u>stearothermophilus</u> or <u>Bacillus</u> <u>licheniformis</u>, the preferred pH is from about 5.5 to about 6.5.

-7-

0182296

In the process of this invention, the granular starch is
treated with the alpha-amylase enzyme preparation for a sufficient
time to solubilize from about 45% to about 90% by weight of the
granular starch. When this portion of starch has solubilized the
residual starch granules have the capacity to sorb from about
1.2 grams to about 2.5 grams of water per gram of starch. If more
than about 90% by weight of the granular starch is solubilized by
enzymatic action, the residual starch tends to have a gritty feel
and be unsuitable as a body dusting starch. In carrying out
the process of this invention, it is preferred to solubilize from
about 66% to about 82% by weight of granular starch.

After the desired amount of the granular starch has been
solubilized by the alpha-amylase enzyme, the remaining porous starch
granules are separated from the reaction mixture. The separation
can be accomplished by any of the conventional means, such as
filtration or centrifugation. At this stage, the porous granular
starch contains some adhering material. If the starch is used
for certain purposes, such as for a body dusting starch, the
color and odor of such adhering materials is undesirable. In
order to remove these materials, the porous granular starch is
washed with a water-soluble organic solvent, such as a lower-
molecular weight alcohol or a lower-molecular weight ketone.

-8-

Examples of suitable solvents for this purpose include methanol, ethanol, isopropanol and acetone. Alternatively, the adhering material can be removed from the porous granular starch by washing with a dilute aqueous solution of a detergent. The dried product is then pulverized to the desired mesh size.

The starch products of this invention can be used as replacement for talc in body dusting powders and in other applications where dusting powders are used. It is often the practice in the formulation of such powders to include some additives to make the powder more acceptable for a particular use. These additives include such materials as fragrances and flow promoters. Such additives may be used with the starches of the present invention and do not interfere with the improved water sorption properties of the starches when used in relatively small concentrations.

The following examples illustrate the invention. It is to be understood that the examples are illustrative only and do not intend to limit the invention in any way.

### EXAMPLE 1

A slurry containing 66 parts of corn starch (dry basis) and 100 parts of water was adjusted to pH 5.7 and held at 60°C for 7 hours. Then 2 units of <u>alpha</u>-amylase enzyme per gram of starch

-9-

was added. The <u>alpha</u>-amylase employed was THERMAMYL, an <u>alpha</u>-amylase enzyme from <u>Bacillus</u> licheniformis, available from Novo Laboratories, Wilton, Connecticut. The mixture was stirred overnight at 60°C before an aliquot of the slurry was removed and filtered. The solid was washed with an equal volume of water and dried. Forty-five percent of the starch was solubilized under these conditions. The insoluble starch granules showed a water sorption of 1.19 grams per gram of starch on a dry solids basis.

Water sorption is determined by weighing accurately about 2 grams of solid in a 40-cc Gooch crucible (Coors) having a perforated bottom covered with a moist Whatman glass microfiber filter. The crucible is covered with a watch glass and set in a petri dish containing a 1% NaCl solution. The depth of the solution in the dish is maintained at about 8 mm. After 24 hours, the crucible is removed, wiped dry and weighed to determine the amount of water sorbed by the solid. For comparison purposes, it was determined that native corn starch sorbs about 0.7 gram of water, and talc sorbs about 0.4 gram of water in this test.

The pH of the remaining portion of the original slurry was readjusted to 5.7 from 5.3 and the mixture was stirred for an additional 6 hours at 65°C. At the end of this period, another aliquot of the reaction mixture was filtered to collect residual granular starch. This material was washed with an equal volume of water and the percent solubilization and water sorption determined as above. In this sample, 49% of the starch had been solubilized and the remaining porous granular starch product sorbed 1.31 grams of water per gram of starch.

To the rest of the original reaction mixture, now at pH 5.3, was added additional THERMAMYL at the rate of 2 units per gram of starch originally present in this portion of the mixture. The mixture was stirred overnight at 65°C before the solid was collected, washed and tested as above. In this case, 55% of the starch originally present in this portion had been solubilized, and the residual porous starch granules sorbed 1.36 grams of water per gram of starch.

-11-

EXAMPLE 2

To 600 ml of a slurry containing 30% by weight on a dry
substance basis of corn starch was added 5.1 ml of 1.0 M $CaCl_2$
solution and the pH was adjusted to 5.5 with 1.0 M $Na_2CO_3$ solution.
The mixture was heated to 50°C before 2 units of THERMAMYL alpha-
amylase enzyme per gram of starch was added. In this example,
0.14 unit of glucoamylase per gram of starch was also added. The
glucoamylase used was a commercial glucoamylase produced by
Aspergillus niger, available as G-ZYME from the Enzyme Development
Company, 2 Penn Plaza, New York, N.Y. The mixture was stirred
while the temperature was raised from 50°C to 65°C over a 90-minute
period. Stirring was continued at 65°C for a total of 21.5 hours
while the pH was maintained at 5.5. The solid was separated by
filtration, washed with methanol and dried in the air. Seventy-four
percent of the starch was solubilized by this process. The insoluble
porous granular starch product sorbed 1.9 grams of water per gram of
starch by the water sorption test described in Example 1. The
product had good tactile properties when used as a body dusting
powder.

-12-

0182296

EXAMPLE 3

The general procedure of Example 2 was repeated using a 25% slurry of starch in water. The starch enzyme mixture was heated from 50°C to 70°C over a 2-hour period and then stirred at 70°C for 22 hours. These conditions caused solubilization of from 82 to 89% of the starch and gave residual porous granular starch products showing water sorption of from 1.9 to 2.3 grams of water per gram of starch.

Thus, it is apparent that there has been provided, in accordance with the invention, dusting powder starches and a process for their preparation that fully satisfy the objects, aims and advantages set forth above. While the invention has been described in conjunction with specific embodiments thereof, it is evident that many alternatives, modifications, and variations will be apparent to those skilled in the art in light of the foregoing description. Accordingly, it is intended to include all such alternatives, modifications, and variations as set forth within the spirit and scope of the appended claims.

-13-

## Claims

1. The process of using a body dusting powder characterized in that the powder comprises porous starch granules which consist essentially of the residue which remains after from about 45% to about 90% by weight of granular starch has been solubilized by an <u>alpha</u>-amylase enzyme preparation.

2. The process of claim 1 characterized in that the granular starch is corn starch.

3. The process of claim 2 characterized in that from about 45% to about 90% by weight of the granular corn starch has been solubilized by agitating an aqueous dispersion of the granular corn starch with the <u>alpha</u>-amylase enzyme preparation at a temperature of from about 60°C to about 75°C and a pH of from about 4.0 to about 7.0.

4. The process of claim 3 characterized in that the <u>alpha</u>-amylase enzyme preparation contains the <u>alpha</u>-amylase produced by <u>Bacillus stearothermophilus</u>.

5. The process of claim 3 characterized in that the <u>alpha</u>-amylase enzyme preparation contains the <u>alpha</u>-amylase produced by <u>Bacillus licheniformis</u>.

-14-

6.    The process of claim 1 characterized in that the porous starch granules are washed with a water-soluble organic solvent.

7.    The process of claim 6 characterized in that the water-soluble organic solvent is selected from the group consisting of methanol, ethanol, isopropanol, and mixtures thereof.

8.    The process of claim 1 characterized in that the porous starch granules are washed with a dilute aqueous solution of a surfactant.

9.    Process for the manufacture of a porous granular starch product by treating granular starch with an alpha-amylase enzyme under conditions which solubilize a portion of the starch while leaving a porous granular starch residue.

10.    A Porous granular starch product capable of sorbing from about 1.2 grams to about 2.5 grams of water per gram of starch and comprising the insoluble residue remaining after granular starch is partially solubilized by an alpha-amylase enzyme.